# EUROPÄISCHE PATENTSCHRIFT

(11) **EP 0 418 639 B1**
(45) Veröffentlichungstag und Bekanntmachung des Hinweises auf die Patenterteilung: **04.01.1995**
(21) Anmeldenummer: 90116944.1
(22) Anmeldetag: 04.09.1990
(51) Int. Cl.: C07D 229/00

(54) **Verfahren zur Herstellung von Uretdiongruppen aufweisenden Polyisocyanaten**
Process for the preparation of uretdion-group containing polyisocyanates
Procédé de préparation de polyisocyanates contenant des groupes uretdiones

(30) Priorität: 14.09.1989 DE 3930670
(43) Veröffentlichungstag der Anmeldung: 27.03.1991
(73) Patentinhaber: BASF Aktiengesellschaft, 67063 Ludwigshafen (DE)
(72) Erfinder: Bruchmann, Bernd, Dr., D-6700 Ludwigshafen (DE)

(56) Entgegenhaltungen:
- EP-A- 0 166 172
- EP-A- 0 317 744
- EP-A- 0 325 941
- EP-A- 0 337 116
- EP-A- 0 377 177
- EP-A- 0 417 603
- SYNTHESIS, Nr. 7, Juli 1975, Seiten 463-464, Stuttgart, DE; R. RICHTER et al.:"Synthesis of dimers and trimers of benzyl isocyanates"

## Beschreibung

Die Erfindung betrifft ein Verfahren zur Herstellung von Uretdiongruppen aufweisenden Polyisocyanaten durch Reaktion von monomeren aromatischen Diisocyanaten in Gegenwart von 1,2-Dimethylimidazol als Katalysator in einem inerten organischen Lösungsmittel.

Es ist grundsätzlich bekannt, Uretdione durch Umsetzung von Isocyanaten in Gegenwart bestimmter Katalysatoren herzustellen.

Aus Richter und Ulrich, Synthesis 1975, 463, ist ein Verfahren bekannt, bei dem Benzylisocyanate in Gegenwart von 1,2-Dimethylimidazol als Katalysator ohne Lösungsmittel umgesetzt werden, wobei jedoch beträchtliche Mengen an Isocyanuraten anfallen. Für eine Reihe von Anwendungen sind Isocyanurate unerwünscht, da sie trifunktionell sind und Zur Vernetzung neigen. Außerdem ist die hier verwendete Katalysatormenge von 10 Gew.-%, bezogen auf das eingesetzte Isocyanat, bezüglich der Wirtschaftlichkeit verbesserungsbedürftig.

Auch bei Noack und Schwetlick, Z. Chem. 26(1), 117 (1986), ist dieser Nachteil der Bildung von Isocyanuraten zu finden. Bei der Reaktion von T80, einem Gemisch aus 80 Gew.-% 2,4- und 20 Gew.-% 2,6-Toluylendiisocyanat mit 1,2-Dimethylimidazol als Katalysator in einer Mischung aus Methylethylketon und Cyclohexan, werden nur 35 % Dimerausbeuten erhalten. Hierbei liegt das Volumenverhältnis von Lösungsmittelgemisch zu Diisocyanat bei 3:1.

Die Verwendung einer Mischung aus verschiedenen Lösungsmitteln ist bei dem technischen Verfahren auch weniger geeignet, da sich die Aufarbeitung der Lösungsmittel aufwendig gestaltet.

Dieser Lehrmeinung entsprechend, beschreibt die EP-A-0 317 744 ein Verfahren zur Herstellung von (cyclo)aliphatischen Uretdionen durch Umsetzung von (cyclo)aliphatischen Diisocyanaten oder Diisocyanatgemischen mit jeweils 6 bis 15 C-Atomen in Abwesenheit von Lösungsmittel und Wasser mit 4-dialkylaminosubstituierten Pyridinen als Katalysator, wobei man das Reaktionsgemisch nach Erreichen eines Dimerisierungsgrades von 10 bis 80 % einer Vakuumdünnschichtverdampfung zur Isolierung des Uretdions unterwirft. Nach Angaben eines Vergleichsbeispiels wird aus Isophorondiisocyanat mit 1,2-Dimethylimidazol als Katalysator nach 10-tägiger Umsetzung bei Raumtemperatur kein Uretdion gebildet.

Nach Angaben der EP-A-0 325 941 können Biuret- und Uretdiongruppen aufweisende Polyisocyanate hergestellt werden durch Umsetzung eines mindestens 15-fachen Überschusses an (cyclo)aliphatischen Diisocyanaten mit 1,5-Diaminoheptanol-6 oder dessen Lösung in bis zu 80 Mol-%, bezogen auf Lösung, Wasser bei 100 bis 210°C und anschließender Entfernung von nicht umgesetztem Diisocyanat.

Die Herstellung der Biuret-, Uretdion- und Isocyanuratgruppen sowie ggf. Allophanatgruppen enthaltenden Polyisocyanate wird vorzugsweise lösungsmittelfrei durchgeführt.

Der Erfindung lag daher die Aufgabe zugrunde, den geschilderten Mängeln abzuhelfen.

Demgemäß wurde ein Verfahren zur Herstellung von Uretdiongruppen aufweisenden Polyisocyanaten durch Reaktion von monomeren aromatischen Diisocyanaten in Gegenwart von 1,2-Dimethylimidazol als Katalysator in einem inerten organischen Lösungsmittel gefunden, wobei das Gewichtsverhältnis von Lösungsmittel zu Diisocyanat im Bereich von 0,1:1 bis 1:1 liegt.

Außerdem wurde gefunden, daß Toluol als Lösungsmittel besonders wirksam ist.

Als monomere aromatische Diisocyanate eignen sich beispielsweise 1,5-Naphthylendiisocyanat 4,4'-Diphenyldiisocyanat, 1,4-Phenylendiisocyanat sowie vorzugsweise 2,6-Toluylendiisocyanat und besonders bevorzugt sind 2,4-Toluylendiisocyanat sowie 4,4'-Diphenylmethandiisocyanat. Grundsätzlich sind jedoch alle aromatischen monomeren Diisocyanate verwendbar. Es versteht sich, daß auch Mischungen von Diisocyanaten eingesetzt werden können.

Das als Katalysator verwendete 1,2-Dimethylimidazol wird vorzugsweise in Mengen von 0,01 bis 10 Gew.-%, bevorzugt 0,05 bis 1 Gew.-%, bezogen auf das eingesetzte Diisocyanat, eingesetzt.

Die bei dem erfindungsgemäßen Verfahren eingesetzten inerten organischen Lösungsmittel sind an sich bekannt. Grundsätzlich eignen sich alle Lösungsmittel, die gegenüber den Ausgangsstoffen, dem Katalysator und den Endprodukten inert sind. Bevorzugt werden Trichlorethylen oder n-Hexan, besonders bevorzugt Toluol verwendet. Das Gewichtsverhältnis des Lösungsmittels liegt im Bereich von 0,1:1 bis 1:1, bezogen auf das Gewicht des Diisocyanats. Bevorzugt wird ein Verhältnis von 0,3:1 bis 1:1 eingesetzt.

Zu dem gelösten Diisocyanat wird vorzugsweise bei Temperaturen von -10°C bis 80°C, bevorzugt bei 20°C bis 50°C unter Rühren der Katalysator, ebenfalls in Lösung, zugesetzt. Das Gewichtsverhältnis des Lösungsmittels liegt im Bereich von 1:1 bis 100:1, vorzugsweise 10:1 bis 60:1, bezogen auf das Gewicht des 1,2-Dimethylimidazols.

Nach einer Reaktionszeit von vorzugsweise 2 bis 48 Stunden, insbesondere 14 bis 21 Stunden bei Temperaturen von vorzugsweise 20 bis :100°C insbesondere 20 bis 50°C, wird das entstandene Umsetzungsprodukt abgetrennt, gewaschen und getrocknet.

Die mit der Erfindung erzielten Vorteile bestehen insbesondere darin, daß der Gehalt an unerwünschtem Isocyanurat kleiner oder gleich 2 % beträgt, und daß man trotz geringer Katalysatorkonzentrationen hohe Ausbeuten an Uretdiongruppen aufweisenden Polyisocyanaten erhält.

Die so hergestellten Uretdiongruppen aufweisenden Polyisocyanate können zur Polyurethanherstellung verwendet werden.

### Beispiele

### Beispiel 1

### Umsetzung von 2,4-Toluylendiisocyanat

500 g 2,4-Toluylendiisocyanat und 200 g Toluol wurden bei 20°C gerührt und mit 2,5 g 1,2-Dimethylimidazol, gelöst in 100 g Toluol, versetzt. Bei dieser Temperatur wurde noch 5 Stunden gerührt und die entstandene Suspension 16 Stunden stehengelassen. Das nun feste Produkt wurde mit Toluol gewaschen und bei 40°C im Vakuum getrocknet.

Zusammensetzung:
95 % Uretdiongruppen aufweisendes Polyisocyanat
4 % nicht umgesetztes 2,4-Toluylendiisocyanat
1 % Isocyanurat

### Beispiel 2

### Umsetzung von 4,4'-Diphenylmethandiisocyanat

Analog Beispiel 1 wurde das gelöste 4,4'-Diphenylmethandiisocyanat bei 50°C mit dem gelösten 1,2-Dimethylimidazol versetzt. Es wurde 2 Stunden bei 50°C gerührt und die Suspension über Nacht bei Raumtemperatur stehengelassen.

Zusammensetzung:
85 % Uretdiongruppen aufweisendes Polyisocyanat
13 % nicht umgesetztes 4,4'-Diphenylmethandiisocyanat
2 % Isocyanurat

## Patentansprüche

1. Verfahren zur Herstellung von Uretdiongruppen aufweisenden Polyisocyanaten durch Reaktion von monomeren aromatischen Diisocyanaten in Gegenwart von 1,2-Dimethylimidazol als Katalysator in einem inerten organischen Lösungsmittel, dadurch gekennzeichnet, daß das Gewichtsverhältnis von Lösungsmittel zu Diisocyanat im Bereich von 0,1:1 bis 1:1 liegt.

2. Verfahren zur Herstellung von Uretdiongruppen aufweisenden Polyisocyanaten nach Anspruch 1, dadurch gekennzeichnet, daß als Lösungsmittel Toluol eingesetzt wird.

3. Verwendung der gemäß den Ansprüchen 1 bis 2 erhaltenen Uretdiongruppen aufweisenden Polyisocyanate zur Herstellung von Polyurethanen.

## Claims

1. A process for the preparation of polyisocyanates containing uretdione groups by reacting monomeric aromatic diisocyanates in the presence of 1,2-dimethylimidazole as catalyst in an inert organic solvent, wherein the solvent:diisocyanate weight ratio is in the range from 0.1:1 to 1:1.

2. A process for the preparation of polyisocyanates containing uretdione groups as claimed in claim 1, wherein the solvent employed is toluene.

3. The use of polyisocyanates containing uretdione groups obtained as described in claims 1 and 2 for the preparation of polyurethanes.

## Revendications

1. Procédé de préparation de polyisocyanates à groupes uretdione par réaction de diisocyanates aromatiques monomères, en présence de 1,2-diméthylimidazole comme catalyseur, dans un solvant organique inerte, caractérisé en ce que le rapport en poids du solvant au diisocyanate est compris entre 0,1/1 et 1/1.

2. Procédé de préparation de polyisocyanates à groupes uretdione selon la revendication 1, caractérisé en ce que l'on utilise comme solvant le toluène.

3. Utilisation des polyisocyanates à groupes uretdione obtenus selon les revendications 1 et 2 pour la préparation de polyurethanes.
